# EUROPEAN PATENT APPLICATION

(11) **EP 4 744 571 A1**
(43) Date of publication of application: **20.05.2026**
(21) Application number: 24212638.1
(22) Date of filing: 13.11.2024
(51) Int. Cl.: A61B 1/00, A61B 1/267

(54) **METHOD FOR OPERATING A VIDEO LARYNGOSCOPE**

(71) Applicant: Kumar, Sujit, Redmond, WA 98052 (US)
(72) Inventor: Kumar, Sujit, Redmond, WA 98052 (US)
(74) Representative: Dr. Gassner & Partner mbB

(57) **Abstract**

The present invention provides a method (300) for operating a video laryngoscope, enhancing usability during endotracheal intubation procedures. The method involves controlling a display unit attached to a laryngoscope handle to present real-time visual data captured by a camera housing located at the distal end of the handle. The display unit automatically adjusts the orientation of the displayed visual data based on the angle of the display unit relative to the handle, ensuring proper alignment during the procedure. The method (300) includes capturing and storing visual data in an integrated memory module, providing clinicians with the ability to review, manage, and capture data through a user-friendly interface. Additionally, the video laryngoscope (100) enables secure connection to external devices for data transfer using password-protected encryption to safeguard patient information. The method (300) improves overall functionality by improving the user experience, enhancing security, and providing clear visual feedback during medical procedures.

## Description

### TECHNICAL FIELD

The present invention relates to the field of medical devices, particularly a video laryngoscope. More specifically, the invention pertains to a video laryngoscope incorporating software-controlled features for real-time image capture, image orientation correction, secure data storage, and data transfer.

### BACKGROUND

Endotracheal intubation is a critical medical procedure often performed to secure a patient's airway during surgeries or in emergency situations. Traditionally, laryngoscopes have been used to aid clinicians in visualizing the airway during intubation. However, conventional laryngoscopes often face challenges, such as limited visibility, the risk of image distortion due to improper lighting, and a lack of integrated features for data management. These limitations are particularly pronounced in patients with high body mass index (BMI) or other anatomical challenges, where the visibility and maneuverability of the device become restricted.

Moreover, modern medical practices demand secure handling of patient data, including the images and videos captured during procedures. Existing devices often lack integrated software capabilities that ensure real-time data capture, secure storage, and seamless access for review. The absence of proper encryption and password protection further increases the risk of unauthorized access to sensitive medical data.

Thus, there exists a need for a video laryngoscope that not only enhances the visual capabilities during intubation but also integrates advanced software features for data management, security, and ease of use.

### STATEMENT OF THE INVENTION

The present invention relates to a method for operating a video laryngoscope that may enhance the endotracheal intubation process by integrating advanced image management, data security, and user interaction functionalities. The method may involve controlling a display unit attached to a laryngoscope handle to present visual data captured by a camera housing located at the distal end of the handle. The display unit may automatically adjust the orientation of the displayed visual data based on the angle of the display unit relative to the handle. The video laryngoscope may capture and store visual data in a memory module within the display unit and provide a user interface allowing users to configure settings, review stored data, and initiate data capture. It may enable a secure connection to external devices for the transfer of visual data utilizing password-protected encryption.

In one aspect, the video laryngoscope may automatically adjust the image orientation when the display unit is rotated beyond predefined angles. The user interface may provide interactive menus accessible through touch gestures, allowing clinicians to adjust brightness, contrast, and audio settings. Visual data may be encrypted with a user-configurable password to secure access and transmission, ensuring patient confidentiality.

The video laryngoscope may include a processor and a memory module for storing visual data related to medical procedures. The display unit may include a touchscreen interface for user interaction, and a software module that may control the capture, display, storage, and transmission of visual data. The software may initiate recording automatically when the laryngoscope is activated, tagging critical moments during the procedure. The video laryngoscope may include multi-factor authentication protocols for accessing stored data and modifying settings, ensuring compliance with healthcare standards for data security.

The video laryngoscope may be configured to communicate wirelessly with external medical equipment and displays, allowing real-time or recorded visual data to be shared securely. The software module may also provide real-time analysis tools to assist clinicians in identifying anatomical structures and obstructions during intubation.

A non-transitory computer-readable medium may store program instructions executable by a processor of the video laryngoscope. The instructions may cause the processor to automatically adjust display settings based on sensor data from the laryngoscope, secure stored data with encryption, and provide a customizable user interface for interaction. The software may allow automatic brightness adjustment based on ambient lighting, notify users when image orientation is corrected, and enable automatic recording and tagging of important moments during procedures.

The video laryngoscope may support cloud-based data management for secure backup and retrieval, enabling remote access by authorized personnel. The program may allow for secure wireless data transfer to external medical devices using encryption protocols, ensuring compliance with healthcare data security standards. Additionally, the video laryngoscope may provide real-time suggestions and alerts based on the visual data captured during intubation, improving clinical accuracy and patient safety.

An object of the present invention is to provide a video laryngoscope with integrated software that enhances the usability of the device by automatically adjusting the orientation of the displayed image based on the position of the display unit during intubation procedures.

Another object is to enable a secure data management system within the video laryngoscope, utilizing password-protected encryption to safeguard stored visual data, ensuring compliance with medical data privacy regulations.

Further, to offer a user-friendly interface on the display unit of the video laryngoscope, allowing clinicians to easily access settings, review stored data, adjust display parameters, and initiate recording through intuitive touch gestures and controls.

Yet another object is to automate the capture and storage of visual data related to endotracheal intubation procedures, with software that can tag significant moments during the procedure for review and analysis.

One more object is to facilitate secure wireless or wired data transfer of recorded images and videos to external devices, such as PCs, tablets, or cloud-based storage systems, while maintaining the highest level of security through encryption and multi-factor authentication.

Further, to provide a real-time image enhancement system, integrated into the software, that adjusts brightness, contrast, and other visual properties dynamically based on feedback from the camera and light sensors.

Also, to integrate cloud-based data management features, enabling remote access, backup, and retrieval of patient data, ensuring that the video laryngoscope complies with healthcare data security standards while providing access to clinical data from anywhere.

One more object is to improve procedural efficiency by offering software that initiates automatic recording upon activation of the laryngoscope and allows clinicians to interact with the video laryngoscope using touch-based controls, enhancing the overall workflow during critical medical procedures.

### BRIEF DESCRIPTION OF THE DRAWINGS

Other features and advantages of the invention will become apparent when reading the detailed description given below, purely by way of example and in a non-limitative manner, referring to the following figures:
FIG. 1 illustrates a side view of a video laryngoscope in accordance with the present invention;
FIG. 2a- 2b illustrate a front view and a top view of a handle of the video laryngoscope in accordance with the present invention;
FIG.3 illustrates a perspective view of a camera housing of the video laryngoscope in accordance with the present invention;
FIG.4 illustrates a perspective view of a deflector of the video laryngoscope in accordance with the present invention;
FIG. 5 illustrates a front view of a display assembly of the video laryngoscope in accordance with the present invention;
FIG.6 illustrates a bottom view of a display cover of the video laryngoscope in accordance with the present invention;
FIG.7a-7b illustrates an image orientation correction feature of the display unit in accordance with the present invention;
FIG.8a-8b illustrates a security mechanism feature of the video laryngoscope in accordance with the present invention;
FIG.9a-9c illustrate a front view of the handle while detaching a blade in accordance with the present invention;
FIG. 10 illustrate a side view of the blade of the video laryngoscope in accordance with the present invention; and
FIG. 11 illustrates a flowchart showing the method of operating the video laryngoscope in accordance with the present invention.

### DETAILED DESCRIPTION

An embodiment of this invention, illustrating its features, will now be described in detail. The words "comprising, "having, "containing," and "including," and other forms thereof, are intended to be equivalent in meaning and be open ended in that an item or items following any one of these words is not meant to be an exhaustive listing of such item or items, or meant to be limited to only the listed item or items.

The present invention provides a video laryngoscope which enhances the performance of medical procedures, particularly endotracheal intubation. The video laryngoscope combines a laryngoscope with integrated software to provide seamless control over image display, data capture, and secure storage. The software automatically adjusts image orientation based on the position of the display unit and provides an intuitive user interface for clinicians to manage settings, review stored data, and capture images or videos during procedures. The video laryngoscope also incorporates security measures, such as password protection and data encryption, to ensure patient data is safely stored and transmitted.

The terms "first," "second," and the like, herein do not denote any order, quantity, or importance, but rather are used to distinguish one element from another, and the terms "an" and "a" herein do not denote a limitation of quantity, but rather denote the presence of at least one of the referenced item. The disclosed embodiments are merely exemplary of the invention, which may be embodied in various forms.

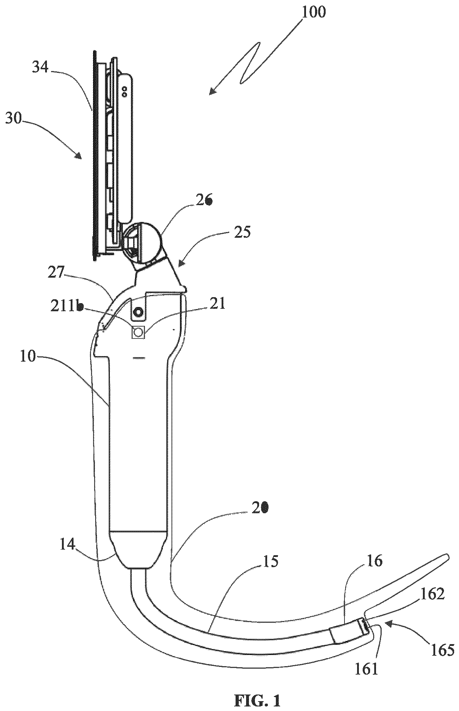

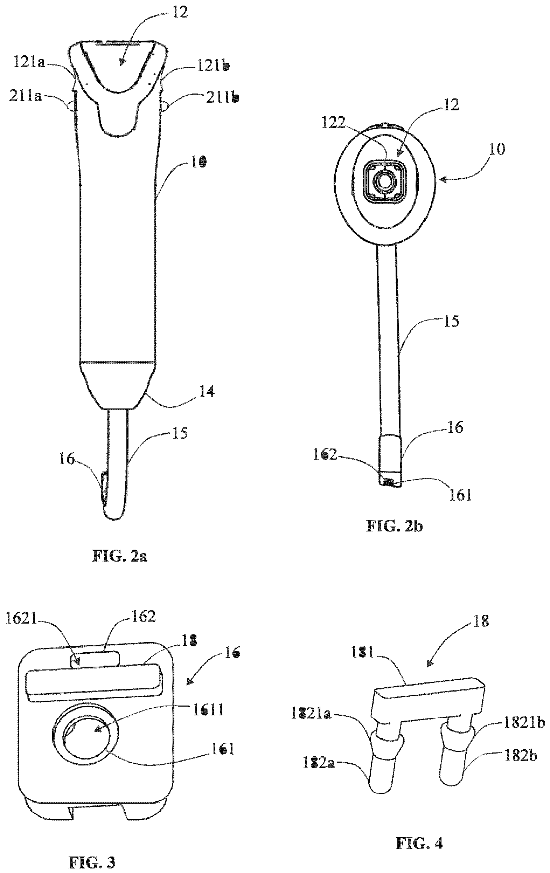

Referring now to FIGs 1, 2a, and 2b, a video laryngoscope (100) in accordance with the present invention is illustrated. The video laryngoscope (100) includes a handle (10), a blade (20), a display unit (30), and an attachment assembly (25) to attach the display unit (30) to the handle (10). The handle (10) is an elongated member that has an ergonomic design that allows clinicians to easily hold the handle (10) during an intubation process. The handle (10) has an upper end and a lower end. The upper end of the handle (10) is provided with a receiving port (12) to receive the attachment assembly (25).

The lower end of the handle (10) is provided with a tapered portion (14). Specifically, the lower end of the handle (10) is provided with a camera channel (15) that extends from the tapered portion (14) of the handle (10). The camera channel (15) is a flexible link extending from the tapered portion (14) of the handle (10) and comprises an electric circuit to transfer the visuals such as images and videos to the receiving port (12). Specifically, the camera channel (15) is electrically connected to a first terminal (122) of the receiving port (12). The camera channel (15) has a first end connected to the tapered portion (14) of the handle (10) and a second end comprising a camera housing (16). The camera housing (16) of the camera channel (15) is provided to capture visuals of the path of the endotracheal tube, larynx, trachea or airway.

Referring now to FIGs 1, and 3, the camera housing (16) comprises an image sensor (161) and a light source (162). The image sensor (161) facilitates capturing of the visuals of the path of the endotracheal tube, larynx, trachea or airway. Specifically, an image sensor opening (1611) in the camera housing (16) allows the image sensor (161) to capture the visuals of the path of the endotracheal tube, larynx, trachea or airway. In the present aspect of the invention, the image sensor (161) is a 2-megapixel (MP) camera, but a person skilled in the art can use any other image sensor (161) having different specifications. For example, a person skilled in the art can configure the image sensor to meet different specifications, such as higher resolution sensors (e.g., 5 MP or 10 MP) for more detailed imaging. The image sensor may also include options for adjusting image brightness, contrast, and zoom functionality for a better visual of the intubation path.

Further, the camera housing (16) includes a light source opening (1621) to allow the light source (162) to illuminate the path of the endotracheal tube and the airway. Furthermore, the camera housing (16) has a deflector (18). Specifically, the deflector (18) is arranged on an outer surface of the camera housing (16) separating the image sensor opening (1611) and the light source opening (1621). The deflector (18) avoids the direct exposure of the light source (162) to the image sensor (161). The light source (162) is configured to provide focused illumination of the path of the endotracheal tube, larynx, and trachea. It can be configured to emit varying intensities of light, depending on the clinical environment or patient anatomy. In particular, the light source is adjustable to illuminate dark or obscured areas, ensuring clear visibility during the intubation process.

In the present aspect of the invention, the deflector (18) is a rectangular strip as shown in FIG 4. The rectangular strip is made up of a silicone material. A person skilled in the art can configure the deflector (18) with any other material, shape or size according to the size and position of the image sensor (161) and the light source (162). The deflector (18) is horizontally arranged between the light source opening (1621) and the image sensor opening (1611) to avoid the scattering of the light on the image sensor (161). Specifically, the deflector (18) is positioned between the light source (162) and image sensor (161) to minimize light scattering that can obscure or distort the visuals captured by the image sensor (161). The deflector is made from a silicone material, known for its light-absorbing properties, but other light-absorbing materials (e.g., polycarbonate or specialized plastic) can also be used. This ensures that the captured images are sharp and free from glare or halos that could obscure the clinician's view.

Referring now to FIGs 3, and 4, the deflector (18) includes a rectangular portion (181), and two legs (182a, 182b) extending from the ends of the rectangular portion (181). The two legs (182a, 182b) are provided with respective locking elements (1821a, 1821b) to attach the deflector (18) to the camera housing (16). Specifically, the camera housing (16) has two openings (not shown) to receive the locking elements (1821a, 1821b) of the two legs (182a, 182b) to hold the deflector (18) on the outer surface of the camera housing (16). The deflector (18) is fixedly attached to the camera housing (16) using the locking elements (1821a, 1821b) but a person skilled in the art can provide the deflector (18) which is detachable from the camera housing (16). Specifically, the deflector (18) is detachable from the camera housing (16) if the deflector (18) has incurred damage or requires replacement.

In another aspect of the invention, the deflector (18) is an extruded portion (not shown) that extends from the outer surface of the camera housing (16). The extruded portion is provided between the image sensor opening (1611) and the light source opening (1621) to avoid direct light exposure to the image sensor (161) from the light source (162). The scattering of the light from the light source (162) makes images distorted and blurry. The deflector (18) avoids direct exposure of the light source (162) to the image sensor (161) to avoid distortion and blurriness of the images.

In another aspect of the invention, the deflector (18) is a thin sheet arranged between the image sensor opening (1611) and the light source opening (1621). The sheet can be made up of any material that absorbs light to avoid the direct exposure of the light source (162) on the image sensor (161).

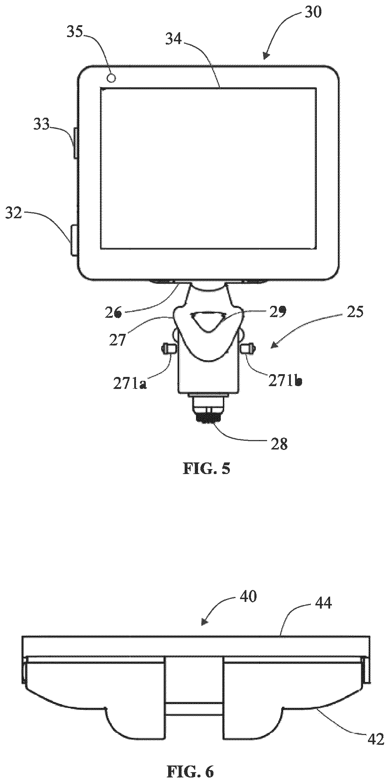

Referring now to FIGs 1, and 5, the display unit (30) is provided which is attachable to the handle (10) through the attachment assembly (25). The attachment assembly (25) includes a housing (27), and a second terminal (28) which is electrically connected to the display unit (30). The housing (27) is connected to the display unit (30) using a hinge (26). The hinge (26) is provided to pivot the display unit (30) in a back-and-forth direction. The hinge (26) is connected to the display unit (30) and the housing (27). Specifically, the hinge (26) has a connecting element (not shown) that is adapted to attach to the housing (27). The housing (27) receives the connecting element of the hinge (26) to allow the display unit (30) to freely rotate around the central axis of the handle (10). The hinge (26) allows the display unit (30) to rotate in all the possible directions to configure the display unit (30) in a required position to provide better visibility to the clinician. The hinge (26) that connects the display unit (30) to the handle (10) allows 360-degree rotation, but it also includes pre-determined stops at angles such as 45, 90, 180, and 270 degrees. These stop points are built into the hinge mechanism, ensuring that the display unit locks into position at these angles. This helps the clinician maintain a stable and optimal view of the airway during intubation, without requiring manual adjustments. The automatic image adjustment ensures that the image remains properly oriented regardless of the display unit's rotation.

Further, the housing (27) includes two ball projections (271a, 271b) extending from an outer surface of the housing (27) and arranged opposite to each other to securely attach and detach the housing (27) to the handle (10). The two ball projections (271a, 271b) are biased to radially extend away from the centre axis of the housing (27). The housing (271a, 271b) is attached to the receiving port (12) of the handle (10). Specifically, the receiving port (12) has two openings to receive the respective ball projections (271a, 271b) of the housing (27) to securely hold the housing (27) within the receiving port (12) of the handle (10). The two ball projections (271a, 271b) ensure that the housing (27) is held in place until the two ball projections (271a, 271b) are pressed to detach the housing (27) of the attachment assembly (25). Specifically, the two ball projections (271a, 271b) have respective balls arranged at the extreme ends to easily press the two ball projections (271a, 271b) while detaching the housing (27) from the handle (10). The two ball projections (271a, 271b) are located on opposite sides of the housing (27). These ball projections are mechanically biased to extend radially outward from the housing (27) and engage with corresponding sockets in the receiving port (12) of the handle (10). This engagement ensures a secure attachment, while pressing the ball projections inward allows for quick detachment of the display unit (30). The ball projections are designed to operate with minimal force, allowing the clinician to quickly adjust or remove the display unit during an intubation procedure

Once the display unit (30) is attached to the handle (10) through the attachment assembly (25), the second terminal (28) of the attachment assembly (25) electrically connects with the first terminal (122) of the receiving port (12) of the handle (10) to establish the electric connection between the camera channel (15) and the display unit (30). Specifically, upon attaching the attachment assembly (25) to the handle (10), the first terminal (122) and the second terminal (28) immediately establish an electric connection between the image sensor (161) and the display unit (30) enabling the instantaneous display of visuals of the path of the endotracheal tube, larynx, trachea, or airway on the display screen (34).

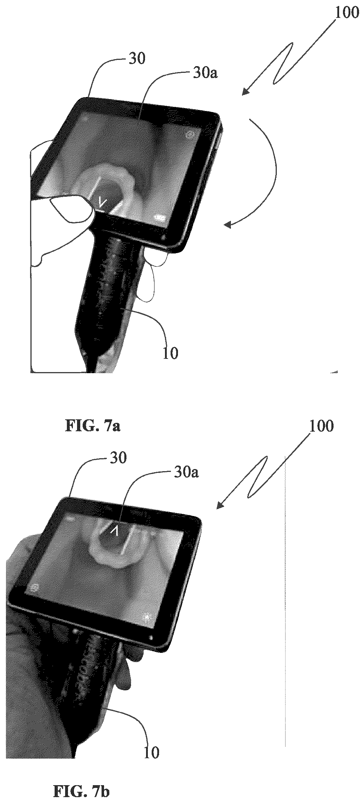

Furthermore, the attachment assembly (25) includes a record button (271) (as shown in figure 5) to capture an image or record a video. In the present aspect of the invention, the record button (271) is arranged on a portion of the housing (27). However, a person skilled in the art can arrange the record button (271) on any other portion in such a way that the clinician's thumb easily reaches to the record button (271) to capture an image or record a video. The display unit (30) further includes an automatic image orientation correction feature (30a) as shown in FIGs7a-7b. When the display unit (30) is rotated downwards or upwards by approximately 180 degrees or other angles, the image on the display screen (34) automatically adjusts to ensure proper alignment for the clinician. This is particularly beneficial in procedures involving high-BMI patients, where the rotated display ensures better visibility and access to the airway.

Referring now to figures 1, 3, and 5, the display unit (30) includes a battery (not shown), a power button (32), an output port (33), a display screen (34), a touch user interface (TUI) (not shown), a processor (not shown), and a memory module (not shown) to store the visuals of the path of the endotracheal tube, larynx, trachea or airway. The video laryngoscope allows clinicians to capture images and videos of the intubation process using the record button arranged on the display unit. The captured data is stored in the integrated memory module. The battery is arranged within the display unit (30) to supply power to the display screen (34), the image sensor (161) and the light source (162). The battery can be a replaceable battery or a rechargeable battery.

In another aspect of the invention, the handle (10) includes a battery (not shown) that can be used to supply power to the image sensor (161) and the light sensor (162) when the display unit (30) is not attached to the handle (10). In such case, the handle (10) establishes a wireless connection with the display unit (30) or an external display (not shown) to transfer the visuals of the path of the endotracheal tube, larynx, trachea or airway from the image sensor (161) to the display unit (30) or the external display.

The power button (32) is provided on any of the sides of the display unit (30). The power button (32) is within the range of the clinician's fingers. In the present aspect of the invention, the power button (32) is arranged at the bottom left of the display unit (30). The power button (32) is used to power on or power off the display screen (34). Specifically, the power button (32) is pressed and released to power on the display screen (34), and the power button (32) is pressed and held for 3-4 seconds to power off the display screen (34). A person skilled in the art can configure the duration for holding the power button (32) to power off the display screen (34). In the present aspect, the power button (32), is located at the bottom left of the display unit (30). A short press activates the display screen (34), image sensor (161), and light source (162), while a longer press (3-4 seconds) powers down the unit. This minimizes accidental shutdowns and ensures that the display is operational when needed. The record button (271) on the housing further allows clinicians to capture visuals during the procedure, with the captured data automatically saved in a secure, password-protected folder.

In another aspect of the invention, the display screen (34) starts displaying the visuals of the path of the endotracheal tube once the display unit (30) is attached to the handle (10) and the display screen (34) is powered ON using the power button (32). The display screen (34) automatically starts displaying the visual of the endotracheal tube without pressing the record button (271).

In one more aspect of the invention, the video laryngoscope (100) is adapted to start displaying the visuals on the display screen (34) even if the display unit (30) is not attached to the handle (10). The display screen (34) displays the visuals by establishing the wireless connection between the handle (10) and the display unit (30).

Further, the output port (33) is provided on any of the sides of the display unit (30) to connect the display unit (30) to an external display (not shown) to show the visuals of the path of the endotracheal tube. The external display has a bigger screen size than the display screen (34) of the display unit (30). It is obvious to a person skilled in the art to provide a plurality of output ports on the display unit (30).

The display screen (34) of the display unit (30) is adapted to show visuals of the path of the endotracheal tube received from the image sensor (161). In the present aspect of the invention, the display unit (30) has a rectangular shape to accommodate the display screen (34). The display screen (34) has a size of 3.5 inches to provide better visibility from all angles. It is obvious to a person skilled in the art to increase the size of the display screen (34) according to the requirement.

Further, the touch user interface is provided to allow the clinician to interact with the video laryngoscope (100). The touch user interface of the display screen (34) is adapted to review and manage previously recorded images or videos which are stored in the memory of the display unit (30). The touch user interface includes a home page to show the current time and date, a menu icon to access the plurality of icons, and a battery icon to indicate the level of the battery. The display screen (34) is powered on using the power button (32) to display the home page. In the present aspect, the touch user interface (TLTI) of the display screen (34) allows the clinician to manually activate the image orientation correction feature (30a) as shown in FIGs 7a and 7b. The image orientation can be flipped by swiping up on the touchscreen (34) or by pressing a dedicated 'flip' button on the display unit (30). The clinician interacts with the menu icon of the home page by touching the menu icon displayed on the display screen (34) to access the plurality of the icons. The menu icon is provided on the bottom left corner of the display screen (34) to allow the clinician to reach the menu icon with one hand. After touching the menu icon, the processor receives the touch input from the touch user interface and processes the command to show the plurality of icons on the display screen (34).

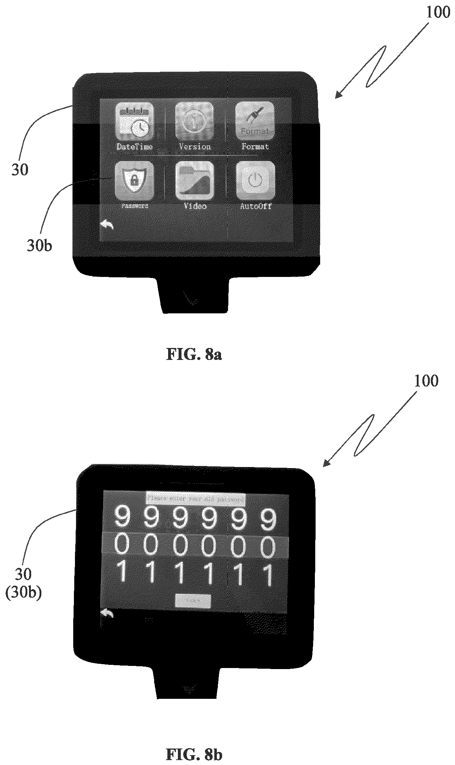

Further, the video laryngoscope (100) includes a security mechanism (30b) for stored visual data, such as images and videos captured during the intubation process as shown in FIGs 8a and 8b. Accessing the stored data on the display unit (30) requires a password, which can be user-defined. The default password is '000000,' but it can be configured to any six-digit combination by the clinician to enhance patient data security. The touchscreen interface on the display unit (30) provides intuitive navigation of the device's settings and stored data. Clinicians can interact with the touch user interface (TUI) using simple gestures, such as swiping or tapping icons, to adjust brightness, access stored images, or activate the image orientation correction feature. The touchscreen is also designed to remain responsive even when the clinician is wearing medical gloves, ensuring seamless operation. The display unit (30) further includes the security mechanism (30b) like an encryption system that protects data during transfer to external devices, such as PCs, tablets, or other computing resources as shown in FIGs 8a and 8b. A password is required not only for accessing the stored data but also for connecting the device to external computing resources, ensuring that sensitive medical data remains secure. The encryption extends to data transfer, ensuring that files sent to external devices such as PCs or tablets are protected by encryption protocols such as AES-256. This level of encryption ensures compliance with healthcare data security standards.

The plurality of icons are assigned with specific actions to perform after receiving the command from the processor of the display unit (30). Specifically, the plurality of icons includes a settings icon to make changes in the brightness of the display screen, the volume of the sound, turn on or turn off the output port (33), and the like. Further, the plurality of icons includes a photo icon to view and manage previously recorded images, and a video icon to view and manage previously recorded videos. In another aspect of the invention, the plurality of icons includes a folder icon to view and manage the previously recorded images and videos in one place. A person skilled in the art can add more icons to the plurality of the icons to provide additional features and functions.

The touch user interface is adapted to show an indication of the captured image on the display screen (34) when the record button (29) is pressed by the clinician to capture the image. Further, the touch user interface is adapted to show a timer of the recording on the display screen (34) when the record button (29) is pressed and held to record the video. Further, the timer disappears from the display screen (34) after pressing the record button (29) to stop the recording.

In another aspect of the present invention, the display unit (30) includes a charging light (35) to indicate the charging status of the battery. The charging light (35) turns on when the battery is connected to a power source to recharge the battery, and turns off when the battery is not connected to the power source for recharging. It is obvious to a person skilled in the art to arrange the charging light (35) in any of the corners of the display unit (30).

Referring now to FIGs 1, 5 and 6, a display cover (40) is provided to prevent the display unit (30) and the attachment assembly (25) from getting infected during the intubation process. The display cover (40) is made up of a transparent material to allow the clinician to view the display screen (34) without any hindrance. The display cover (40) includes a lower panel (42) and an upper panel (44) hinged at a hinge point. The lower panel (42) and the upper panel (44) pivot around the hinge point to cover the display unit (30). The upper panel (44) and the lower panel (42) of the display cover (40) are pressed to snap fit with each other after placing the display unit (30) within a cavity of the display cover (40). Further, the upper panel (44) has a flexible sheath (not shown) extending from the free end of the upper panel (44) to cover the attachment assembly (25) and a portion of the handle (10). The display cover (40) is disposable and can be disposed of once the intubation process is finished.

Referring again to FIGs 1, and 2, the handle (10) is adapted to receive the blade (20) from the lower end of the handle (10). The blade (20) is received around the outer surface of the handle (10) to entirely cover the handle (10) and the camera channel (15). The blade (20) is attached to the handle (10) while performing the intubation process. The handle is adapted to receive all types of blades like Macintosh, and Miller. Further, the handle (10) is adapted to receive both adult blades and child blades. The camera channel (15) is adapted to receive all types of blades like Macintosh, and Miller for both adults and children. The handle (10) does not require any other adjustments or mountings to receive adult blades and child blades. The blade (20) is slidable over the camera channel (15) to attach with the handle (10) to perform the intubation process. The blade (20) is detachably attached to the handle (10) and is detachable from the handle (10) after the intubation process.

In the present aspect of the invention, the blade (20) is attachable to the handle (10) through a dual ball mechanism (21) of the handle (10). The dual ball mechanism (21) includes two ball projections (211a, 21 1b) arranged opposite to each other to securely attach and detach the blade (20) to the handle (10). The two ball projections (211a, 21 1b) are biased to radially extend away from the centre axis of the handle (10). The two ball projections (211a, 211b) engage with the blade (20). Specifically, the blade (20) has two openings (not shown) to engage with two ball projections (211a, 211b) of the handle (10) to securely hold the blade (20) thereover. The two ball projections (211a, 21 1b) ensure that the blade (20) is held in place until the two ball projections (211a, 211b) are pressed to detach the blade (20). The blade (20) is disposed of after detaching from the handle (10) to reduce the chance of infection and cross-contamination. Disposing of the blade (20) after one use allows the video laryngoscope (100) to control the spread of infection which can be spread by using the same blade again and again.

Further, the blade (20) has an opening (165) to allow the image sensor (161) and the light source (162) to capture the visuals of the path of the endotracheal tube. The image sensor (161) captures the visuals of the path of the endotracheal tube, larynx, trachea or airway and transfers the visuals to the display unit (30).

Before performing the intubation process, the clinicians attach the blade (20) to the handle (10). The blade (20) can be an adult blade or a child blade. The blade (20) is slidable over the camera channel (15) and is attached to the handle (10) through the dual ball mechanism (21) of the handle (10).

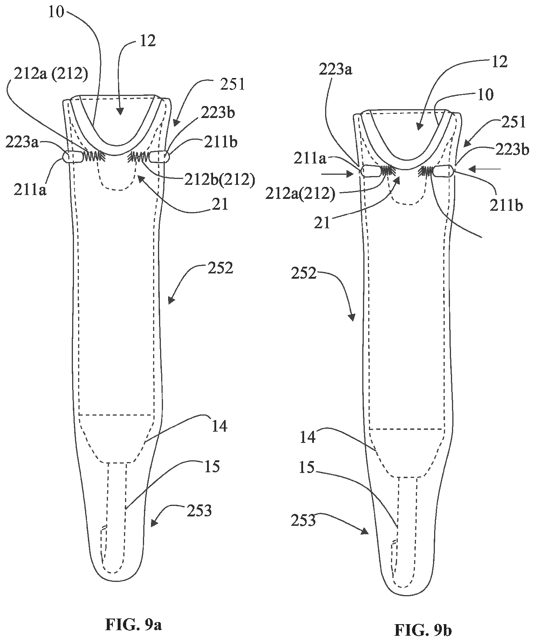

Referring now to FIGs. 9a-9c, in the present embodiment of the invention, the blade (20) is attachable to the handle (10) through an attachment mechanism (21) of the handle (10). The attachment mechanism (21) includes at least two ball projections (211a, 211b) arranged opposite to each other to securely attach and detach the blade (20) to the handle (10). The two ball projections (211a, 211b) are biased to radially extend away from the centre axis of the handle (10). The two ball projections (211a, 211b) are configured to engage with the blade (20). Specifically, the two ball projections (211a, 211b) are biased to extend radially outward from the center of the handle (10) using a biasing member (212) such as springs. The biasing member (212) is positioned inside the handle (10) and applies a continuous force to the ball projections (211a, 211b), to configure an extended position of two ball projections (211a, 211b) during the attachment of the blade (20). The two ball projections (211a, 21 1b) have corresponding biasing members (212a, 212b) to bias the two ball projections (211a, 211b) away from the handle (10) to engage and hold the blade (20) in an attached position. When the blade (20) is attached, the ball projections (211a, 211b) engage with corresponding openings (223a, 223b) in the blade (20), holding the blade (20) around the handle (10).

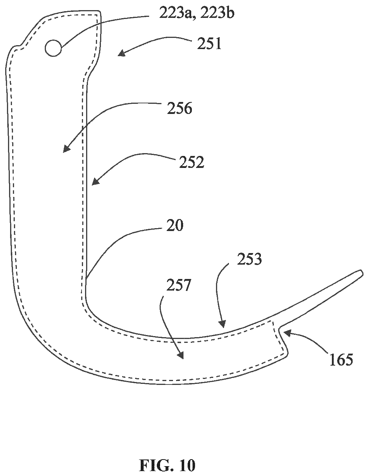

Referring now to FIG. 10, the blade (20) includes an attaching portion (251), a gripping portion (252), and a tongue portion (253). The attaching portion (251) is at the proximal end of the blade (20) and is adapted to attach the blade (20) to the handle (10). The attaching portion (251) includes at least two openings (223a, 223b) positioned to engage with the two ball projections (211a, 211b) of the handle (10). The two openings (223a, 223b) are provided to engage with the corresponding two ball projections (211a, 21 1b) during the intubation process. The attachment mechanism (21) allows the detachment of the handle (10) by pressing the two ball projections (211a, 211b) inward. The blade (20) has a first cavity (256) formed at the attaching portion (251) and the gripping portion (252) of the blade (20). The first cavity (256) is having a shape that is adapted to fit over the handle (10) and cover the handle (10) during the intubation process. The first cavity (256) allows the blade (20) to accommodate the handle (10) and remain firm in place during the intubation process. The first cavity (256) is formed at the attaching portion (251) and extends through the gripping portion (252).

The gripping portion (252) is a middle section of the blade and is arranged between the attaching portion (251) and the tongue portion (253). The gripping portion (252) is configured to provide a grip for the clinician during the intubation process. The gripping portion (252) has indentations (not shown) or other surface textures on the outer surface of the gripping portion (252) to enhance the grip of the clinician.

Furthermore, the tongue portion (253) is a distal end of the blade (20) located after the gripping portion (252). The tongue portion (253) is provided to interact with the patient's anatomy, particularly during intubation. In the present embodiment, the tongue portion (253) is an integral part of the blade (20) and transversally extends from the gripping portion (252). The tongue portion (253) is configured to receive the camera channel (15) and the camera housing (16). The tongue portion (253) includes a camera housing opening (165) to accommodate the camera housing (16) facing outside of the blade (20) allowing an image sensor (161) arranged within the camera housing (16) to capture visuals of the patient's airway, including the endotracheal tube, larynx, and trachea.

Specifically, the blade (20) has a second cavity (257) formed at the tongue portion (253) of the blade (20). The second cavity (257) is configured to accommodate the camera channel (15) and the camera housing (16) therein. The second cavity (257) accommodates the camera channel (15), which runs along the length of the tongue portion (253). The second cavity (257) provides space for the camera channel (15) to be housed within the blade (20). Further, the camera housing (16) is exposed through the camera housing opening (165) arranged in the tongue portion (253) to allow the image sensor (161) to capture the visuals of the patient's airway.

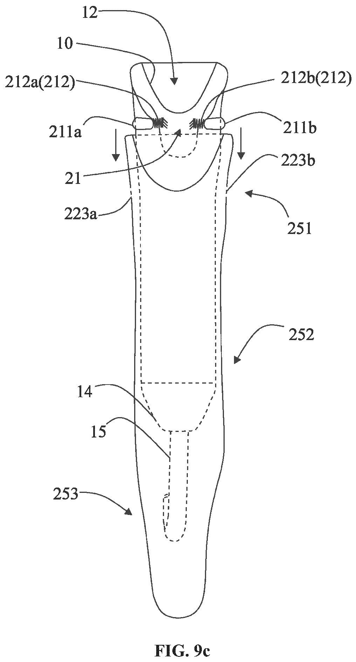

After the intubation process, the blade (20) is detachable from the handle (10) by pressing the two ball projections (211a, 211b) against the biasing force exerted by the biasing members (212a, 212b). When the pressure to the two ball projections (211a, 211b) is applied, the two ball projections (211a, 211b) are pushed inward, overcoming the biasing force of the biasing members (212a, 212b) and moving toward the center of the handle (10) (as shown in FIG. 9b). The two ball projections (211a, 21 1b) are moved towards the center of the handle (10) to disengage from the corresponding openings (211a, 21 1b) in the blade (20), allowing the blade (20) to be detached from the handle (10). Particularly, upon pressing the two ball projections (211a, 211b), the blade (20) is slidable over the handle (10) and the camera channel (15) in a downward direction (shown in FIG. 9c), thereby completely removing the blade (20) from the handle (10) and disposing of it after the intubation process.

Further, the display unit (30) is placed inside the lower panel (42) of the display cover (40) and the upper panel (44) is pivoted to close the display cover (40). The flexible sheath is wrapped around the attachment assembly (25) and the portion of the handle (10). Once the flexible sheath is wrapped, the video laryngoscope (100) is ready to use for the intubation process. The display unit (30) is rotated to configure the position according to the clinician's requirement. The movement of the display unit (30) is facilitated by the hinge (26).

While performing the intubation process, the power button (32) is pressed to activate the display screen (34), the image sensor (161), and the light source (162). The visuals of the path of the endotracheal tube and the airway are displayed on the display screen (34). The deflector (18) of the camera housing (16) reduces the scattering of the light from the light source (162). The deflector (18) helps to reduce the distortion and blurriness of the images. Further, the record button (271) is provided to capture the image and record the video. The record button (271) is pressed and released to capture the image, and the record button (271) is pressed and held for 2 to 3 seconds to record the video. The video laryngoscope (100) is completely covered from the outside eliminating the risk of the video laryngoscope (100) getting infected. After the intubation process, the display cover (40) is removed from the display unit (30) and disposed of to avoid spreading infection. Further, the blade (20) is removed from the handle (10). The blade (20) which is mainly in contact with the body parts is disposed of after one use which helps to reduce the rate of infection or cross-contamination spread due to the repetitive use of the blade (20).

Further, the video laryngoscope (100) allows the clinician to have one hand free while operating the video laryngoscope (100) during the intubation process. In an operating room, clinicians need free hands to simultaneously use multiple devices or perform multiple actions. With the placement of the power button (32) at the bottom left of the display unit (30), the menu icon on the bottom left of the display screen (34) and the record button (29) under the display unit (30), the clinician can fully operate the video laryngoscope (100) with their left hand thus leaving the right hand free.

In another aspect of the present invention, the video laryngoscope (100) includes a handle (10), a blade (20), and a display unit (30). The display unit (30) is attached to the handle (10) without using an attachment assembly (25). Specifically, the display unit (30) includes an attaching element (not shown) extending from a bottom portion of the display unit (30) to attach with the handle (10). It is obvious to a person skilled in the art to configure the attaching element of the display unit (30) to provide a threaded attachment or a snap-fit attachment or a similar attachment, to ensure a secure and functional connection between the display unit (30) and the handle (10).

Further, the video laryngoscope (100) includes a software module providing instructions that control the capture, display, storage, and transmission of visual data. The software module automatically initiates the recording of the procedure when the laryngoscope is activated and tags specific moments in the recording based on user input or automated criteria. The software module provides real-time analysis tools to assist in the identification of anatomical structures and potential obstructions during intubation

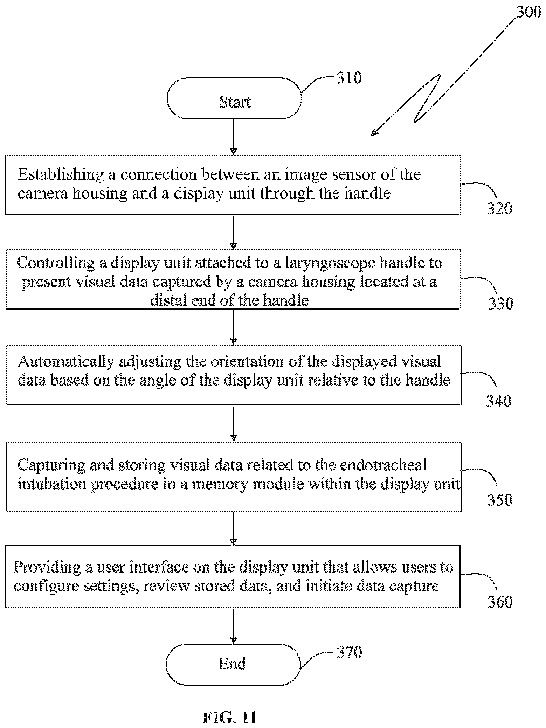

In an aspect as shown in FIG 11, a computer implemented method (300) for operating a video laryngoscope in accordance with the present invention is provided. The video laryngoscope integrates software that improves its overall functionality, particularly in terms of image management, data security, and user interaction. The software is executed by an embedded processor within the display unit (30), which controls the various operations of the video laryngoscope.

The method (300) starts at step 310.

At step 320, the display unit (30) is attached to the handle (10) to establish a connection between the image sensor (161) and the display unit (30) through the handle (10). The connection is an electrical connection between the image sensor (161) and the display unit (30) through the camera channel (15) and the handle (10).

At step 330, The display unit (30) attached to the handle (10) is controlled to present visual data captured by a camera housing (16) located at the distal end of the handle (10). The display unit (30) functions as the primary interface between the clinician and the visual data generated during intubation procedures. The camera housing (16), positioned at the distal end of the handle (10), contains an image sensor (161) that captures real-time visuals of the patient's airway. These visuals are useful for guiding the clinician during the insertion of an endotracheal tube into the trachea.

The display unit (30), attached to the handle (10), is adapted to present these captured visuals to the clinician in real-time. The connection between the display unit (30) and the camera housing (16) enables the clinician to view the pathway of the intubation, ensuring that the endotracheal tube is properly positioned within the airway.

As the image sensor (161) captures visual data, the software processes and transmits this data to the display unit (30). The display unit (30) provides a clear, high-resolution image with minimal latency, ensuring that the visuals are accurate and immediate, which is useful for maintaining safety during the procedure.

The attachment mechanism between the display unit (30) and the handle (10) allows for 360-degree rotation, ensuring that the display can be adjusted to the most convenient position for the clinician, without interrupting the display of the visual data.

At step 340, djusting the orientation of the displayed visual data automatically based on the angle of the display unit (30) relative to the handle (10). The software manages the display of real-time visual data captured by the image sensor (161). In the present embodiment, orientation sensors are included within the laryngoscope (100) to detect the angle of the display unit (30) relative to the handle (10) and adjust the image accordingly. For instance, if the display unit (30) is rotated beyond 90° or 180°, the system automatically flips the image to ensure proper alignment. This is useful during procedures involving patients in challenging positions, as it maintains clear, upright visuals for the clinician.

Further at step 350, The visual data related to the endotracheal intubation procedure is captured and stored in a memory module within the display unit (30). The system allows clinicians to capture images and videos of the intubation process using a record button (29) arranged on the display unit (30). The captured data is stored in an integrated memory module. The software also allows for the automatic tagging of critical moments during the procedure, based on user inputs or predefined criteria, such as the detection of anatomical landmarks.

Further, at step 360, The clinicians can navigate through stored data using the touchscreen interface (TUI) of the display unit (30), allowing them to review, delete, or tag important visuals for later reference. This feature is helpful for post-procedural review, documentation, and data management, enhancing the overall utility of the device in clinical settings. Once the intubation procedure is complete, the video laryngoscope (100) allows clinicians to access and review the stored visuals. These may include both real-time images captured during the procedure and recorded videos. In an aspect, the TLTI displays the files in an organized manner, for example, sorted chronologically or tagged by specific events, making it easy for clinicians to locate the exact moment they need to review.

In an aspect, using the TUI, clinicians can apply tags or labels to any significant moments, making it easier to retrieve them later. The tagging feature is customizable, allowing clinicians to use predefined tags (such as "difficult airway," "anatomical marker," or "tube placement") or create their own custom tags relevant to the specific procedure.

In an aspect, the TUI is configured to be intuitive and responsive, even when the clinician is wearing medical gloves, ensuring seamless operation during or after the procedure. The interface supports touch gestures like swiping to navigate through visual files, tapping to select and open images or videos, and pinching to zoom in on important details.

The method ends at step 370.

Further, the system video laryngoscope (100) enables a secure connection to external devices for the transfer of visual data utilizing password-protected encryption. The video laryngoscope (100) has the capability to securely transfer visual data, such as images and videos captured during medical procedures, to external devices including computers, tablets, or external medical displays. This functionality allows for secure access to patient data while maintaining data integrity and compliance with healthcare data protection standards. The video laryngoscope (100) provides a password-protected encryption mechanism, ensuring that sensitive medical information is protected from unauthorized access during both storage and transmission.

Upon initiating a connection to an external device, the video laryngoscope (100) requires the user to enter a password to authenticate access. The video laryngoscope (100) includes a default password configuration, which may be user-defined for enhanced security. In one embodiment, the video laryngoscope (100) requires a six-digit password to unlock access to stored data or to initiate a data transfer to external devices. This prevents unauthorized personnel from accessing sensitive patient information.

Additionally, the video laryngoscope (100) can be configured to support multi-factor authentication (MFA) for heightened security. In this configuration, access to data and the ability to transfer visual data to external devices is restricted by requiring both a password and a secondary authentication factor. For instance, MFA may include biometric authentication, such as fingerprint scanning or facial recognition, or the use of a time-based one-time passcode (OTP) sent to a registered mobile device. This added layer of protection ensures that only authorized users can access or transfer the stored visual data.

Once the user has been authenticated, the visual data is encrypted prior to transfer. In an exemplary embodiment, the video laryngoscope (100) utilizes Advanced Encryption Standard (AES) with 256-bit encryption, which is a widely accepted encryption method in the healthcare industry. AES-256 encryption ensures that even if the data is intercepted during transfer, it cannot be decrypted or accessed by unauthorized parties.

The encrypted data is then transmitted to external devices via both wired and wireless methods. In one embodiment, the video laryngoscope (100) supports wired connections, such as USB or HDMI, for data transfer to computers or larger display systems. In another embodiment, the video laryngoscope (100) supports wireless communication protocols, including Wi-Fi or Bluetooth, enabling real-time transfer of data to external medical equipment or displays.

In a wireless configuration, the video laryngoscope (100) can connect to external medical displays or hospital servers using password-protected encryption to ensure the security of patient data during transmission. This feature allows the clinician to display real-time visual data on larger screens or share it with other medical professionals during complex procedures. In another embodiment, the video laryngoscope (100) supports cloud-based storage, allowing for remote backup of visual data. The data transferred to the cloud is encrypted using the AES-256 encryption to protect patient privacy and ensure compliance with healthcare data regulations.

The video laryngoscope (100) also allows secure retrieval of cloud-stored data by authorized personnel. Access to this data requires password authentication or, where applicable, multi-factor authentication.

In a preferred embodiment, during or after a procedure, the clinician initiates a secure data transfer by selecting the appropriate function on the touchscreen interface. For instance, after completing an intubation, the clinician may wish to transfer the captured video data to a hospital's electronic medical record (EMR) system. The video laryngoscope (100) prompts the user to enter the password, and upon successful authentication, the video file is encrypted and securely transmitted to the EMR system over a secure network connection.

In an exemplary use case, a clinician uses the video laryngoscope (100) to capture real-time visual data during an intubation procedure. Once the procedure is completed, the clinician initiates a data transfer to the hospital's server to store the visual data as part of the patient's medical records. The video laryngoscope (100) prompts the clinician to enter a password to authenticate access. Once the password is verified, the visual data is encrypted and securely transmitted to the server using AES-256 encryption, ensuring that the data is protected throughout the transfer process.

If the clinician requires access to this data from a remote location, the cloud-based storage option allows authorized personnel to retrieve the encrypted data by entering the appropriate password or passing multi-factor authentication. This secure connection ensures that only authorized users can access the patient's sensitive medical information.

The software also includes customization options that allow clinicians to create shortcuts for frequently used features, streamlining their workflow. For example, shortcuts can be created for quick access to image orientation correction, brightness settings, or reviewing captured data.

In a preferred embodiment, the video laryngoscope (100) is used during the intubation of a high-BMI patient. The clinician attaches a Macintosh blade (20) to the handle (10) using the dual ball mechanism (21). The clinician rotates the display unit (30) by 180 degrees to avoid interference with the patient's chest, and the video laryngoscope (100) automatically adjusts the image orientation, maintaining an upright and clear view.

The clinician presses the record button (271) to capture the intubation process. The software tags critical moments, such as the tube's placement, based on predefined criteria. After the procedure, the clinician accesses the captured data through the TLTI and reviews the tagged moments. The data is securely transferred to a hospital database using encrypted wireless communication, ensuring the patient's data is protected.

In another aspect, a non-transitory computer-readable medium storing program instructions executable by a processor of the video laryngoscope (100) in accordance with the present invention is provided.

The program instructions enable the processor (not shown) to automatically adjust the display settings based on sensor data from the laryngoscope. This feature ensures that the image remains upright and properly aligned, regardless of the display's position. An on-screen indicator provides visual confirmation when the image orientation is adjusted.

The video laryngoscope (100) may include ambient light sensors that adjust the brightness and contrast of the display automatically, ensuring optimal visibility under various lighting conditions, including dimly lit or brightly illuminated environments.

To protect the visual data, the program includes encryption mechanisms that protect stored data from unauthorized access. Multi-factor authentication (MFA), such as a password and biometric input, is required to access stored data or connect to external devices. This ensures compliance with data security standards in healthcare.

The video laryngoscope (100) provides a customizable user interface that allows clinicians to create shortcuts for frequently used features, enhancing the system's usability during medical procedures. These shortcuts are configurable directly through the touchscreen.

The program instructions automatically initiate the recording of visual data when the laryngoscope is activated. Important/critical moments during the procedure are tagged based on predefined criteria or user input, ensuring efficient review and analysis later.

The video laryngoscope (100) supports wireless data transfer to external devices, ensuring secure communication through encrypted protocols. The video laryngoscope (100) also provides cloud-based backup for visual data, allowing remote access and retrieval by authorized personnel.

In some embodiments, the software includes real-time analysis tools that assist clinicians by providing suggestions or alerts based on visual data. These suggestions may include identifying anatomical landmarks or warning of potential obstructions during intubation.

Therefore, the present invention has the advantage of providing the video laryngoscope (100) to perform the intubation process. The video laryngoscope (100) is provided with the display unit (30) which rotates and moves in the back-and-forth direction to increase the visibility for the clinician. Further, the handle (10) of the video laryngoscope (100) is adapted to receive the adult blades as well as child blades, which reduces the other requirements like different handles for different blades or mountings to receive the different types of blades on the same handle (10). Further, the deflector (18) reduces the distortion and blurriness of the images for better quality visualisation. Furthermore, the handle (10) is covered by the blade (20) and the display unit (30) is covered by the display cover (40) which completely covers the video laryngoscope (100) and prevents the video laryngoscope (100) from getting infected during the intubation process. The video laryngoscope (100) ensures that the captured visuals are automatically adjusted based on the angle of the display unit relative to the handle. This eliminates the need for manual adjustments, allowing clinicians to maintain clear and upright visuals during intubation, particularly beneficial for patients in challenging positions or with high BMI. Further, the clinicians can easily capture and store visual data, such as images and videos, using the intuitive interface. The video laryngoscope automatically tags critical moments during procedures based on predefined criteria or user input, improving the organization of procedural records and facilitating post-procedural analysis.

The foregoing descriptions of specific embodiments of the present invention have been presented for purposes of illustration and description. They are not intended to be exhaustive or to limit the present invention to the precise forms disclosed, and obviously, many modifications and variations are possible in light of the above teaching. The embodiments were chosen and described in order to explain the principles of the present invention best and its practical application, to thereby enable others skilled in the art to best utilise the present invention and various embodiments with various modifications as are suited to the particular use contemplated. It is understood that various omission and substitutions of equivalents are contemplated as circumstance may suggest or render expedient, but such are intended to cover the application or implementation without departing from the scope of the claims of the present invention.

## Claims

1. A method (300) for operating a video laryngoscope (100), the method (300) comprising the steps of:
establishing a connection between an image sensor (161) of the camera housing (16) and a display unit (10) through the handle (10) when the display unit (30) is attached to the handle (10);
controlling the display unit (30) to present visual data captured by a camera housing (16) located at a distal end of the handle (10);
automatically adjusting the orientation of the displayed visual data based on the angle of the display unit (10) relative to the handle (10);
capturing and storing visual data related to the endotracheal intubation procedure in a memory module within the display unit (30);
providing a user interface on the display unit (30) that allows users to configure settings, review stored data, and initiate data capture.

2. The method (300) as claimed in claim 1, wherein the method includes enabling a secure connection between the video laryngoscope and external devices for the transfer of visual data utilizing password-protected encryption.

3. The method (300) as claimed in claim 1, wherein adjusting the orientation of the displayed visual data includes an automatic mode that corrects the image orientation when the display unit (30) is rotated beyond predetermined angles relative to the handle (10).

4. The method (300) as claimed in claim 1, wherein the user interface provides interactive menus accessible through touch gestures, allowing clinicians to adjust brightness, contrast, and volume levels of alerts and playback sounds.

5. The method (300) as claimed in claim 1, further comprising encrypting the visual data using a user-configurable password that must be entered to access or transmit the stored data.

6. A video laryngoscope (100) comprising:
a processor;
a memory module for storing visual data related to medical procedures;
a touchscreen display unit for user interaction;
a software module providing instructions that control the capture, display, storage, and transmission of visual data;
a security mechanism that restricts access to stored data and controls connections to external devices using password protection and encryption protocols.

7. The video laryngoscope (100) as claimed in claim 6, wherein the software module automatically initiates the recording of the procedure when the video laryngoscope (100) is activated and tags specific moments in the recording based on user input or automated criteria.

8. The video laryngoscope (100) as claimed in claim 6, wherein the security mechanism includes multi-factor authentication protocols for accessing the video laryngoscope and modifying its settings.

9. The video laryngoscope (100) as claimed in claim 6, wherein the software module provides real-time analysis tools to assist in the identification of anatomical structures and potential obstructions during intubation.

10. A non-transitory computer-readable medium storing program instructions executable by a processor of a video laryngoscope (100), the instructions when executed cause the processor to:
adjust display settings automatically based on sensor data indicating the display unit's orientation;
secure data storage through encryption and access the stored data via a secure authentication method;
provide a customizable user interface that allows medical personnel to interact with various software features directly from the display unit (10), enhancing usability during medical procedures.

11. The non-transitory computer-readable medium as claimed in claim 10, wherein the program instructions enable the display unit (30) to provide an on-screen indicator that notifies the user when the image orientation has been automatically adjusted.

12. The non-transitory computer-readable medium as claimed in claim 10, wherein the program instructions cause the processor to initiate recording of visual data when the video laryngoscope (100) is activated, without requiring user input, and automatically tag important moments during the intubation procedure based on predefined criteria.

13. The non-transitory computer-readable medium as claimed in claim 10, wherein the secure authentication method comprises a multi-factor authentication process that requires both a password and a secondary authentication factor such as a biometric input or a time-based one-time passcode (OTP).

14. The non-transitory computer-readable medium as claimed in claim 10, wherein the program instructions enable wireless data transfer to external medical devices or displays, and utilize encryption protocols to secure the data during transmission, ensuring compliance with healthcare data security standards.

15. The non-transitory computer-readable medium as claimed in claim 10, wherein the program instructions enable the display unit (30) to provide real-time suggestions or alerts to the clinician based on the visual data, such as identifying anatomical landmarks or obstructions during the intubation process.
